# EUROPEAN PATENT SPECIFICATION

(11) **EP 0 577 565 B1**
(45) Date of publication and mention of the grant of the patent: **04.11.1998**
(21) Application number: 93830187.6
(22) Date of filing: 30.04.1993
(51) Int. Cl.: G01N 33/576

(54) **Stabilized compositions of the delta antigen peptide of hepatitis D virus**
Delta-Antigen-Peptid des Hepatitis-D Virus enthaltende stabilisierte Zusammensetzungen
Compositions stabilisées contenant un peptide antigénique delta du virus de l'hepatite D

(30) Priority: 08.06.1992 IT RM920430
(43) Date of publication of application: 05.01.1994
(73) Proprietor: WABCO B.V., 7940 KB Meppel (NL)
(72) Inventor: Barbieri, Ugo, I-13040 Saluggia (VC) (IT); Borla, Maurizio, I-13040 Saluggia (VC) (IT)
(74) Representative: de Simone, Domenico

(56) References cited:
- EP-A- 0 251 575
- EP-A- 0 485 347
- WO-A-91/06562
- JOURNAL OF IMMUNOLOGY vol. 125, no. 1 , July 1980 , BALTIMORE US pages 318 - 324 M. RIZZETTO ET AL. 'The hepatitis B virus-associated delta antigen: isolation from liver, development of solid-phase radioimmunoassays for delta antigen and anti-delta and partial characterization of delta antigen.'
- PATENT ABSTRACTS OF JAPAN vol. 7, no. 130 (P-202)(1275) 7 June 1983 & JP-A-58 048 855 (MIDORI JUJI K. K.)

## Description

This invention relates to stabilized compositions of the delta antigen peptide (HDAg) of the Hepatitis D Virus (HDV), or antigenic portions thereof, to be used either for storage of such peptide or for the preparation of immuno-diagnostic kits.

HDV expression and replication are hepatitis B virus HBV- dependent; HDV is a human patogenic agent, which is transmitted together with HBV.

The coding sequence of the HDAg antigen is disclosed by Wang, K.S. and al. "Sciences", 1986, 323, 508-513. The diagnosis of HDV infection is performed by means of immuno-assays, like ELISA or RIA, able to detect anti-HDAg immunoglobulins or HDAg peptides in the serum (Crivelli, O. and al., J. of Clin. Microbiol., 1981, 14, 1173-177; Fields, H.A. and al. Am. J.Pathol., 122, 308-314; Shattock, A.G. and al. J. Med. Virol.,1984, 22, 323-331).

A recombinant polypeptide with HDAg antigenic activity was disclosed in the European Patent Application EP 0485347; the peptide is coded by the recombinant plasmid pSORIN δ (DSM N 6774).

Both recombinant and natural HDAg peptides, the latter obtained from HDV infected chimpanzee or marmot hepatic tissues, show a very poor stability after purification. Therefore, many problems arise when said peptides are to be used for immuno-assays and for the preparation of commercial diagnostic kits, for hospitals and laboratories.

Therefore it is evident the need to provide stabilized compositions of HDAg, or HDAg-equivalent peptides, or portions thereof with an HDAg antigenic activity, to be used for the preparation of diagnostic kits for immuno-assays to detect anti-HDV antibodies or HDAg peptides in human biological fluids.

The authors have obtained a stabilized composition of said peptides, by resuspending them under acidic pH conditions.

Rizzetto M. et al. (1980) Joumal of Immunology, 125, 318-324 showed that HDAg is stable at pH 2.4. However the publication neither shows the HDAg stability over long period of time nor the feasibility of an immunoenzymatic assay at such strong acidic pH conditions.

Therefore, an object of the present invention is a stabilized composition of HDAg or HDAg-equivalent peptides, or portions thereof with an HDAg antigenic activity, comprising the fact that said peptides are resuspended in an aqueous solution with a pH between 3.0 and 6.5, preferably between 3.5 and 6.0. Preferably said acidic pH is established by the presence of ions at a concentration between from 0.001 M and 0.05 M; more preferably between 0.005 M and 0.02 M. According to a preferred embodiment of this invention, said ions comprise either citrate, phosphate or acetate ions.

A further object of the invention are immuno-diagnostic kits for the in vitro detection of anti-HDAg antibodies in samples from biological fluids comprising as specific ligand the HDAg or HDAg-equivalent peptides, or portions thereof with an HDAg antigenic activity in a stabilised acid buffered form according to the invention.

In a preferred embodiment, said kits comprise a solid carrier coated with the composition according to the invention, said coating occurring by incubating said composition with said solid carrier in an aqueous solution with an acidic pH, preferably between 5 and 6.

According to a preferred embodiment, said HDAg or HDAg-equivalent peptides, or portions thereof with an HDAg antigenic activity are biotinilated.

This invention is described in the following Examples, for explicative but not limitative purposes.

### Example 1 Detection Assay of Anti-HDV IgMs

A solid carrier, consisting of microtiter well plates, is pre- treated with anti-human IgM antibodies, according to procedures known by those skilled in the art. Samples from human sera are added to wells incubated for 1 hr at 37°C. The plate is then washed and different dilutions of (horse radish peroxidase) HRP-conjugated polyclonal (or monoclonal) anti-HDV antibodies are added in a pH 7 buffer solution, in order to keep the pH of the reaction mix as closest as possible to neutral values; a HRP-conjugated monoclonal antibody, specifically recognizing the WRRYRRH HDAg amino acid sequence, is advantageously used.

A B-HDAg-T peptide (EP 0485347) at a precalculated concentration is added in 10 mM, pH 4 citrate/phosphate buffer.

After 1-2 hours at 37°C, the reagents are removed by washing and HRP sensitive substrate and a dye are added, such as H₂O₂ and TMB (tetramethylbenzoine) and blocked after 30 minutes by 1N sulphuric acid. The optical density is measured on a photospectrometer at 630 and 450 nm wavelengths. The difference of OD₄₅₀ - OD₆₃₀ values is directly proportional to the IgM amount in the serum.

The stability, after a 7 day storage both at room temperature and at 4°C, of the B-HDAg-T polypeptide dissolved in a 0.01 M, pH 4 phosphate/citrate buffer or in pH 7.4 phospate buffer is shown in table 1.

**Table 1**

| | pH 4.0 | | pH 7.4 | |
|---|---|---|---|---|
| Sera | 4°C | 37°C | 4°C | 37°C |
| sp1 | 1.307 | 1.235 | 1.465 | 0.934 |
| sp2 | 0.682 | 0.641 | 0.764 | 0.516 |
| sp3 | 0.256 | 0.238 | 0.295 | 0.195 |
| sn1 | 0,036 | 0.038 | 0.041 | 0.055 |
| sn2 | 0.048 | 0.050 | 0.056 | 0.064 |
| sp: positive IgM anti-HDAg serum | | | | |
| sn: negative serum. | | | | |

### Example 2: Detection of Total Anti-HDV Igs

A solid carrier consisting of a microtiter plate wells, is pre-treated with anti-HVD polyclonal (or monoclonal) antibodies; a monoclonal antibody specifically recognizing the WRRYRRH sequence may be used effectively. An aqueous solution of the B-HDAg-T peptide (EP 0485347) at a 0.05 µg/ml concentration is added.

Alternatively, the B-HDAg peptide is biotinylated that was added at 0.01 µg/ml concentration to the insoluble carrier, previously sensitized with streptavidin. After the aspiration of the unbound reagent, the plate is incubated for 1 hour at room temperature with a solution of 0.01 M phosphate buffer, 4% polyvinilpyrrolidone and 10 % polydestrose, ph 5.5, able to stabilize the recombinant protein. After the solution aspiration and the drying having been completed, the plate is ready for the assay.

The serum to be tested is added together with 100 µl of a determined amount of one of the named anti-HDV antibodies, conjugated with horse radish peroxidase (HRP).

After having been incubated from 1 to 3 hours, the plates are washed out and a substrate and a dye, both sensitive to peroxidase activity, are added; for inst., a H₂O₂/tetramethylbenzidine mixture. The reaction is then stopped after 30 minutes by adding 1N sulphuric acid. The optical density is measured on a photospectrometer at 630 and 450 nm wavelenghts. The difference of OD₄₅₀ - OD₆₃₀ values is inversely proportional to total Igs in the serum.

The table 2 herebelow shows the OD values that resulted from plates mantained seven days at 37°C and stabilized with buffers at pH 5.5 and 7.2.

**Table 2**

| | pH 5.5 | | pH 7.2 | |
|---|---|---|---|---|
| Serum | 4°C | 37°C | 4°C | 37°C |
| CN | 0.906 | 1.171 | 1.283 | 1.004 |
| CP | 0.008 | 0.007 | 0.007 | 0.007 |
| sp1 | 0.170 | 0.191 | 0.168 | 0.118 |
| sp2 | 0.432 | 0.484 | 0.526 | 0.340 |
| sn1 | 0.702 | 0.802 | 1.182 | 0.944 |
| sn2 | 1.091 | 0.839 | 0.984 | 0.751 |
| CN: negative control; CP positive control; sp: positive anti-HDAg serum; sn: negative serum | | | | |

## Claims

1. A stabilized composition of HDAg or HDAg-equivalent peptides, or portions thereof with an HDAg antigenic activity, wherein said peptides are suspended in an aqueous acid solution having a pH between 3.0 and 6.5.

2. A stabilized composition of HDAg or HDAg-equivalent peptides, or portions thereof with an HDAg antigenic activity according to the claim 1, wherein said aqueous acid solution has a pH between 3.5 and 6.0.

3. A stabilized composition of HDAg or HDAg-equivalent peptides, or portions thereof with an HDAg antigenic activity according to any previous claims, wherein said acidic pH is established by the presence of ions at a concentration between from 0.001 M and 0.05 M.

4. A stabilized composition of HDAg or HDAg-equivalent peptides, or portions thereof with an HDAg antigenic activity according to according to claim 3, wherein said acidic pH is established by the presence of ions at a concentration between 0.005 M and 0.02 M.

5. A stabilized composition of HDAg or HDAg-equivalent peptides, or portions thereof with an HDAg antigenic activity according to the claims 3 or 4, wherein said ions comprise either citrate, phosphate or acetate ions.

6. Immuno-diagnostic kits for the in vitro detection of anti-HDAg antibodies in samples from biological fluids comprising as specific ligand the HDAg or HDAg-equivalent peptides, or portions thereof with an HDAg antigenic activity in a stabilised acid buffered form according to any of previous claims.

7. Immuno-diagnostic kits for the in vitro detection of anti-HDAg antibodies in samples from biological fluids according to claim 6 comprising a solid carrier coated with the composition according to any of claims from 1 to 5.

8. Immuno-diagnostic kits for the in vitro detection of anti-HDAg antibodies in samples from biological fluids according to claim 7 wherein said coating occurs by incubating said composition with said solid carrier in an aqueous solution with an acidic pH.

9. Immuno-diagnostic kits for the in vitro detection of anti-HDAg antibodies in samples from biological fluids according to claim 8 wherein acidic pH is between 4 and 5.

10. Immuno-diagnostic kits for the in vitro detection of anti-HDAg antibodies in samples from biological fluids according to claim 9 wherein said HDAg or HDAg-equivalent peptides, or portions thereof with an HDAg antigenic activity are biotinilated.

## Patentansprüche

1. Stabilisierte Zusammensetzung von HD-AG- oder HD-AG-Äquivalent-Peptiden, oder deren Teile mit HD-AG-Wirkung, worin die vorgenannten Peptide in einer wässrigen Säurelösung mit pH zwischen 3,0 und 6,5 aufgeschwemmt sind.

2. Stabilisierte Zusammensetzung von HD-AG- oder HD-AG-Äquivalent-Peptiden, oder deren Teile mit HD-AG-Wirkung nach Anspruch 1, worin die vorgenannte wässsrige Säurelösung einen pH-Wert zwischen 3,5 und 6,0 aufweist.

3. Stabilisierte Zusammensetzung von HD-AG- oder HD-AG-Äquivalent-Peptiden, oder deren Teile mit HD-AG-Wirkung nach je einem der vorhergehenden Ansprüche, worin der säurige pH-Wert durch Anwesenheit von Ionen mit einer Konzentration zwischen 0,001 M und 0,05 M bestimmt wird.

4. Stabilisierte Zusammensetzung von HD-AG- oder HD-AG-Aquivalent-Peptiden, oder deren Teile mit HD-AG-Wirkung nach Anspruch 3, worin der vorgenannte säurige pH-Wert durch Anwesenheit von Ionen mit einer Konzentration zwischen 0,005 M und 0,02 M bestimmt wird.

5. Stabilisierte Zusammensetzung von HD-AG- oder HD-AG-Äquivalent-Peptiden, oder deren Teile mit HD-AG-Wirkung nach Anspruch 3 oder 4, worin die vorgenannten Ione aus Zitrat-, Phosphat- oder Azetat-Ionen bestehen.

6. Immunodiagnostik-Kit zur in vitro -Ermittlung von Anti-HD-AG-Antikörpern in Proben aus biologischen Flüssigkeiten, die als spezifische Binder die HD-AG- oder HD-AG-Äquivalent-Peptide, oder deren Teile mit einer HD-AG-Wirkung in einer stabilisierten säuregepufferten Form nach je einem der vorhergehenden Ansprüche enthalten.

7. Immunodiagnostik-Kit zur in vitro -Ermittlung von Anti-HD-AG-Antikörpern in Proben aus biologischen Flüssigkeiten, nach Anspruch 6, enthaltend einen festen, mit der Zusammensetzung gemäss je einem der Ansprüche 1 bis 5 überzogenen Träger.

8. Immunodiagnostik-Kit zur in vitro -Ermittlung von Anti-HD-AG-Antikörpern in Proben aus biologischen Flüssigkeiten nach Anspruch 7, worin der vorgenannte Überzug durch Inkubation der vorgenanten Zusammensetzung mit dem Träger in einer einen säurigen pH-Wert aufweisenden wässrigen Lösung erhalten wird.

9. Immunodiagnostik-Kit zur in vitro -Ermittlung von Anti-HD-AG-Antikörpern in Proben aus biologischen Flüssigkeiten nach Anspruch 8, worin der säurige pH-wert zwischen 4 und 5 liegt.

10. Immunodiagnostik-Kit zur in vitro -Ermittlung von Anti-HD-AG-Antikörpern in Proben aus biologischen Flüssigkeiten nach Anspruch 9, worin die vorgenanten HD-AG- oder HD-AG-Aquivalent-Peptide oder der Teile mit einer HD-AG-Wirkung biotimiert sind.

## Revendications

1. Composition stabilisée des HD-Ag peptides ou HD-Ag équivalentes peptides, ou leur portions avec une HD-Ag antigénique activité, dans laquelle lesdits peptides sont suspendus dans une solution acide acqueuse ayant un pH entre 3.0 et 6.5

2. Composition stabilisée des HD-Ag peptides ou HD-Ag équivalentes peptides, ou leur portions avec une HD-Ag antigénique activité selon la revendication 1, dans laquelle ladite solution acide acqueuse a un pH entre 3.5 et 6.0

3. Composition stabilisée des HD-Ag peptides ou HD-Ag équivalentes peptides, ou leur portions avec une HD-Ag antigénique activité selon l'une quelconque des revendications précédentes, dans laquelle ledite pH acide est determiné par la présence des ions à une concentration entre 0.001M et 0.05 M.

4. Composition stabilisée des HD-Ag peptides ou HD-Ag équivalentes peptides, ou leur portions avec une HD-Ag antigénique activité selon la revendication 3, dans laquelle ledit pH acide est determiné par la présence des ions à une concentration entre 0.005 M et 0.02 M.

5. Composition stabilisée des HD-Ag peptides ou HD-Ag équivalentes peptides, ou leur portions avec une HD-Ag antigénique activité selon la revendication 3 ou 4, dans laquelle lesdits ions consistent des ions de citrates, phosphates ou acetates.

6. Kit immuno-diagnostique pour la détection in vitro des anticorps des anti HD-antigènes des fluides biologiques, comprenant, comme liant specifique, des HD-Ag peptides ou HD-Ag équivalentes peptides ou leur portions ayant une HD-Ag activité, dans une forme stabilisée tamponée par acide, selon l'une quelconque des revendications précédetes.

7. Kit immuno-diagnostique pour la détection in vitro des anticorps des anti HD-antigènes des fluides biologiques selon la revendication 6, comprenant un véhicule solide, revêtu de la composition selon l'une quelconque des revendications 1 à 5.

8. Kit immuno-diagnostique pour la détection in vitro des anticorps des anti HD-antigènes des fluides biologiques selon la revendication 7, dans laquel ledit revêtement a lieu par l'incubation de ladite composition, avec ledit véhicule solide, dans une solution acqueuse ayant un pH acide.

9. Kit immuno-diagnostique pour la détection in vitro des anticorps des anti HD-antigènes des fluides biologiques selon la revendication 8, dans lequel le pH acide est comprise entre 4 et 5.

10. Kit immuno-diagnostique pour la détection in vitro des anticorps des anti HD-antigènes des fluides biologiques selon la revendication 9, dans laquel lesdits HD-Ag peptides ou HD-Ag équivalentes peptides, ou leurs portions ayant une activité HD-antigènique sont biotinés.
